# EUROPEAN PATENT APPLICATION

(11) **EP 3 701 993 A1**
(43) Date of publication of application: **02.09.2020**
(21) Application number: 19159723.6
(22) Date of filing: 27.02.2019
(51) Int. Cl.: A61M 25/00, A61M 27/00, A61M 25/01

(54) **URINARY CATHETER ASSEMBLY WITH INTEGRATED PIVOTABLE HANDLE**

(71) Applicant: Dentsply IH AB, 431 21 Mölndal (SE)
(72) Inventor: FOLENIUS, Isak, 413 13 GÖTEBORG (SE)
(74) Representative: AWA Sweden AB

(57) **Abstract**

A catheter assembly comprises a casing (2) having a tubular compartment, a cap sub-assembly (3) and a catheter (1) having a catheter shaft. The distal part of the catheter is pivotally connected to the cap sub-assembly by a joint. In a closed storage position, the catheter shaft is accommodated in the tubular compartment and the cap sub-assembly seals the open end of the tubular compartment, wherein the catheter shaft extends aligned in the longitudinal direction of the casing and of the cap sub-assembly. The cap sub-assembly, in an open use position, is attachable to the casing with the catheter in an angularly displaced position, in which the longitudinal direction of the catheter is at an angle to the longitudinal direction of the casing and of the cap sub-assembly, the casing thereby serving as a laterally extending gripping member for the catheter.

## Description

### Technical field of the invention

The present invention relates to a catheter assembly, and preferably a urinary catheter assembly. The invention is particularly related to hydrophilic catheters, and specifically to hydrophilic urinary catheters. The invention is also related to methods for preparing such an assembly for use.

### Background

The present invention relates to a urinary catheter assembly. Urinary catheters are commonly used for draining urine from the bladder. Urinary catheters can be of an indwelling type, for long term use, such as days or even weeks, or for intermittent use, whereby the catheters are used for a single draining procedure, typically lasting a few minutes. Intermittent urinary catheters are e.g. used by a large group of persons for self-catheterization, which is a daily-life procedure, taking place several times a day. Typically, catheters for intermittent catheterization are used by patients suffering from urinary retention, due to e. g. spinal cord injury, Multiple Sclerosis or Prosthatic Hyperplasia. Using an intermittent catheter, the bladder may be drained through a natural or artificial urinary canal. Many catheters, such as those for intermittent catheterization, are provided with a hydrophilic coating or the like, providing a smooth and slippery surface when wetted, for safe and comfortable insertion in the urinary canal.

Individuals who suffer from urinary incontinence will normally self-catheterize several times a day. Self-catheterization involves removing the catheter assembly from its package and inserting and advancing the catheter tube through the user's urethra. However, preparation and manipulation of known catheter assemblies is often complicated and tedious. Further, often users of intermittent urinary catheters have limited or diminished dexterity, e.g. as a result of spinal cord injuries. Also, users of intermittent catheters are often required to self-catheterize outside the privacy of the home, such as in public restrooms. Thus, for these and other reasons, it is desirable that the intermittent catheters are provided in discrete packaging that is easy to open and manipulate, which are compact and portable, and wherein the catheter can be deployed and used in a way that alleviates concerns about inadvertent urine leakage or spillage and avoids pain or discomfort to the user.

Many attempts to obtain this have been made in the past. For example, WO 03/002179 proposes a catheter assembly where a handle may be positioned over the catheter shaft in a storage position, and extending away from the catheter shaft in a use position. Similarly, US 2016/0067445 discloses a catheter assembly where a handle is rotatably connected to the rear end of a catheter shaft, so that it can be folded in over the catheter for storage, and folded out for use. US 6653700 discloses a similar arrangement. However, all of these known assemblies are relatively difficult to use and prepare for use, and are also relatively complicated and costly to produce.

Thus, there is still a need for improved urinary catheter assemblies. The assembly should preferably be relatively simple and cost-efficient to produce. Further, the assembly should be easy and intuitive to open and use, even for users with reduced dexterity. The assembly should also preferably be rather small, so that it can easily be carried around by the user in his/her daily life, and easily and discretely be discarded after use. Thus, the assembly is preferably compact to minimize its volume, thereby facilitating transportation, storage, travel, etc.

### Summary of the invention

It is therefore an object of the present invention to provide a catheter assembly which at least alleviates the above-discussed problems.

This object is obtained by means of a catheter assembly in accordance with the appended claims.

According to a first aspect of the invention there is provided a catheter assembly comprising:
a casing having a tubular compartment which is closed at one end and open at the other end;
a cap sub-assembly;
a catheter having a catheter shaft, a proximal insertion end and a distal part having a distal end, wherein a flow path is defined between the proximal end and the distal end, and wherein the distal part is pivotally connected to the cap sub-assembly by a joint, and preferably a revolute joint;
wherein, in a closed storage position, the catheter shaft is accommodated in the tubular compartment and the cap sub-assembly seals the open end of the tubular compartment, wherein the catheter shaft extends in a longitudinal direction aligned with a longitudinal direction of the casing and of the cap sub-assembly; and
wherein the casing is provided with a first engagement member, and the cap sub-assembly is provided with a second engagement member, whereby the cap sub-assembly, in an open use position, is attachable to the casing with the catheter in an angularly displaced position, in which the longitudinal direction of the catheter is at an angle to the longitudinal direction of the casing and of the cap sub-assembly, the casing thereby serving as a laterally extending gripping member for the catheter.

In the present application, the term "proximal" is used to indicate the end or portion of a catheter that is inserted into the body of the user, i.e. the end or portion of the catheter that during use is closer in proximity to the user's body and/or initially enters the user's body upon insertion. The term "distal" is used to refer to an end or portion of the catheter that is opposite the proximal end or portion and is typically further away from the user's body. For the sake of consistency, when the terms "distal" and "proximal" are used in the context of other components, such as the cap sub-assembly and the tubular casing, which are not intended for introduction into the user's body. For such other components, "proximal" refers to the end or portion that is closer to the proximal end of the catheter when the catheter is housed within the assembly, while "distal" refers to an end or portion located opposite to such proximal end or portion.

In accordance with the present invention, the casing serves several purposes. It functions as a package, for maintaining at least the insertable part of the catheter in a clean and sterile condition during storage, prior to use. When the catheter is a hydrophilic catheter, the casing may also accommodate a wetting fluid for activation of the hydrophilic catheter, and preferably for maintaining the catheter continuously wetted and activated during storage. However, alternatively, wetting fluid may be accommodated in a separate container or compartment within the casing, to be released prior to use. The package can also be resealable, allowing the catheter to be reinserted into the package after use, and preferably to be closable once the catheter has been reinserted. However, the casing also functions as a handle for allowing convenient and effective gripping and handling of the catheter during use, which is of particular importance for users having reduced or poor dexterity. Hereby, a relatively large gripping area is provided, allowing the user to use a multifold various positions and techniques for handling the catheter. A user may e.g. grip the handle provided by the casing with the entire hand, in a palmar grip, a position which is very useable in particular for users having poor dexterity. However, the handle may also be gripped by the use of only the fingers, such as two or three fingers, e.g. in a traditional pencil grip, a drumstick grip, a paint brush grip, a cross thumb grip or the like. Thus, the handle is very versatile, allowing gripped in any suitable position.

Further, the casing is relatively long. The casing preferably has an elongate shape, and preferably has a length which at least exceeds the length of the insertable part of the catheter shaft. The catheter assembly may be for female use. The female urethra is normally about 5 cm long, and thus the length of the insertable part of the catheter shaft is preferably 4-10 cm long. Correspondingly, the casing preferably has a length in the range of 5-15 cm, and preferably in the range 8-12 cm. Due to the relatively long length of the casing, the user may choose whether to grip the handle close to the catheter, or some distance away from the catheter. This is particular advantage, since users may have difficulty in reaching too far down. Further, many users would also prefer to handle the catheter from above, without e.g. having to reach down below the rim of the toilet bowl.

This function is further enhanced by the angularly displaced position of the handle, i.e. the casing, so that it extends laterally from the catheter.

Further, for users and situations where an enlarged handle is not necessary, the catheter may still be used without attaching the cap sub-assembly to the casing. This makes the new catheter assembly even more versatile.

Thus, the catheter assembly provides a compact and neat storage position. In this position, the catheter assembly could easily be carried around discretely, e.g. in a handbag, in a pocket or the like, without attracting notice. Further, due to the engagement members, the catheter assembly can easily be transformed into the use position, in which the handle extends out laterally, for convenient use, as discussed in the foregoing. After use, the catheter assembly may be directly discarded. However, it may alternatively be returned to the closed position, so that the used catheter assembly can then be carried by the user in the same discrete way as the unopened assembly, and without the risk of spillage etc, to be discarded later.

The angular position between the catheter and the casing, in the open use position, may be one or several fixed positions, in which the catheter may be fixed or secured. However, the catheter and the casing may alternatively be freely rotatable in relation to each other within an angular range, and fixedly or securable in any such positions. Preferably, the angular position(s) is/are in the range exceeding 0 degrees and up to 120 degrees. Preferably, the angular range is 10-120 degrees, and preferably 30-120 degrees, and more preferably 45-110 degrees, and even more preferably 60-100 degrees, and most preferably 70-100 degrees, such as 85-95 degrees. In one embodiment, the angular position is about 90 degrees. In some embodiments, the catheter may also remain moveable also during use.

In one embodiment, the catheter, in the open use position, is at an angle to the longitudinal direction of the casing and cap sub-assembly in the range of 60-120 degrees, and most preferably 75-105 degrees.

The cap sub-assembly or the distal part of the catheter, or both, may also be provided with notches or the like, loosely maintaining the catheter in the angled position. Hereby, the catheter will be maintained in the angled position once it has been arranged in this state, and unless the catheter by a mild force is pivoted into a different position. This facilitates assembling into the second use position, since the catheter will remain in the angled position during the connection of the cap sub-assembly and the casing.

The connection between the cap sub-assembly and the casing may effect further locking of the catheter in the pivoted, angled position, thereby ensuring that it is maintained in this position during use. This may e.g. be obtained by compression of the cap sub-assembly around the catheter in the use position.

The casing and the cap sub-assembly preferably have aligned and parallel longitudinal directions both in the closed storage position and the open use position.

The catheter assembly is also cost-effective to produce, since it comprises relatively few, and easily producible parts. For example, the casing and cap sub-assembly may be produced by extrusion, injection molding or the like. Further, the catheter may be identical or similar to a conventional catheter, e.g. having a relatively straight inner lumen, etc. This allows the catheter to be produced in a conventional way, and thereafter be attached or included into the assembly. For example, the relatively straightly occurring inner lumen of the catheter, and the avoidance of any obstacles at or in the vicinity of the output area, i.e. close to the distal end, enables manufacturing elements, such as rods and the like, to be inserted into the catheter from the rear end during production. This is of great advantage e.g. when applying a coating to the catheter surface, when punching out drainage openings, thermoforming of the tip, etc.

The catheter assembly is preferably elongate, and preferably having an essentially circular cross-sectional shape. However, other shapes, such as a rectangular or oval cross-sectional shape are also feasible. The overall appearance of the catheter assembly may e.g. be similar to that of a pen.

The cap sub-assembly is preferably removably attached to the casing, providing a sealed enclosure within the casing for at least the insertable part of the catheter during storage, thereby maintain the insertable part of the catheter in a clean and sterile state. The cap sub-assembly is removed from the casing upon use, but is preferably reattachable to the casing after use, thereby again providing a sealed compartment.

The catheter may e.g. be a urinary catheter. The catheter preferably comprises one or several drainage openings, so-called eyes or eyelets, arranged at or in the vicinity of the proximal insertion end. A part of, or the whole, catheter shaft may form an insertable part or insertable length of the catheter. At least the insertable part may further be provided with a hydrophilic coating, or in other ways been provided with a hydrophilic surface, which exhibits a lowered friction when wetted. Further, the distal part may, at least on a part thereof, have larger cross-sectional dimensions than the catheter shaft. The distal part may e.g. be flared or funnel shaped, increasing in dimension towards the distal end, thereby enabling connection of a tube, collection bag or the like.

The second engagement member arranged on the cap sub-assembly may be provided in various positions.

In one embodiment, the second engagement member is provided on an end of the cap sub-assembly being inserted into the opening of the casing in the closed position. Such an insertable second engagement member may, in the open position, be attached to a first engagement member arranged on various positions on the casing, such as on the inner or outer surface of the open end of the casing, or on an inner or outer surface of the closed end of the casing.

In an alternative embodiment, the second engagement member is arranged on a part of the cap sub-assembly which extends outwardly over and away from the opening of the casing in the closed position. Such an outwardly extending second engagement member may, in the open position, be attached to a first engagement member arranged on various positions on the casing, such as on the inner or outer surface of the open end of the casing, or on an inner or outer surface of the closed end of the casing.

In yet another alternative embodiment, the second engagement member is arranged on an inner or outer surface of the end of the cap sub-assembly being opposite to the casing. Such a second engagement member may, in the open position, be attached to a first engagement member arranged on various positions on the casing, such as on the inner or outer surface of the open end of the casing, or on an inner or outer surface of the closed end of the casing.

The first engagement member provided on the casing may be provided in various positions, as already exemplified above. For example, the first engagement member may be provided as an inner or outer surface in the vicinity of the open end of the casing. Further, the first engagement member may be provided as an outer surface of the closed end of the casing. However, the casing may also comprise an inward protrusion at the closed end, such as an extending outer rim, a tubular recession or the like, and consequently, the first engagement member may also be provided on an inner surface of the closed casing end.

The first and second engagement members are arranged to attach the cap sub-assembly with the casing in such a way that the parts do not inadvertently fall apart. In other words, the first and second engagement members, when engaged, resist separation to a certain degree, thereby maintaining the parts together until deliberately brought apart.

The first and second engagement members are preferably complementary to each other in shape and/or size, thereby enabling engagement between the members. For example, one of the members may be slightly convex and the other slightly concave, or the members may both be slightly conical or tapered.

In one embodiment, the first engagement member comprises a tapered, generally conical surface arranged at or in the vicinity of the end of the casing.

One of the first and second engagement members are further preferably shaped and sized so that it may be inserted into the other of the first and second engagement members, so that the engagement member is formed as an outwardly facing surface on the insertable, male part, and as an inwardly facing surface on the receiving, female part.

The first and second engagement members may form a friction fit connection. In a preferred embodiment, the first and second members may comprise tapered, generally conical surfaces, having a corresponding shape, and arranged to be attached by means of a friction fit. Alternatively, or additionally, the members may be arranged to be connected to each other by means of mechanical engagement, such as through a threading, a snap lock arrangement, or the like.

According to an embodiment, the first and second engagement members are connectable through one of threads, a friction fit, a bayonet mount, a Luer connection, a snap-lock and a snap-fit arrangement.

The catheter is pivotally connected to the cap sub-assembly by a joint, and preferably a revolute joint. The revolute joint may also be referred to as a pin joint or hinge joint. The revolute joint is a one-degree-of-freedom joint, providing a single-axis rotation function.

In one embodiment, the distal part of the catheter is provided with laterally protruding pins, and the cap sub-assembly is provided with corresponding openings accommodating the protruding pins, thereby pivotally connecting the catheter to the cap sub-assembly. Thus, here, the revolute joint is formed by laterally protruding pins arranged on the distal part of the catheter and corresponding openings for accommodation of the protruding pins on the cap sub-assembly. However, alternatively, the cap sub-assembly may be provided with laterally protruding pins, extending into corresponding openings of the distal part of the catheter. However, other types of revolution joints are also feasible, as would be appreciated by the skilled reader.

The cap sub-assembly may comprise a head portion providing a sealed closure of the case, and an insertable part being insertable into the casing, the insertable part being pivotally connected to the distal part of the catheter.

The entire catheter may be accommodated in the casing, or in the casing and the cap sub-assembly.

The catheter may be a hydrophilic catheter, having a hydrophilic surface. The hydrophilic surface may be arranged as a hydrophilic coating arranged on a substrate of the catheter, as is per se well known in the art. However, the hydrophilic surface may alternatively be arranged as an integrated part of the catheter, such as an integrated layer, or alternatively, the entire catheter, or part(s) of the catheter, may be made of a hydrophilic material. The hydrophilic surface is preferably arranged to provide low friction when wetted.

In an embodiment of the invention the catheter shaft is arranged to be inserted into a body cavity or body passageway, and the shaft presents the hydrophilic surface on an exterior surface thereof. The hydrophilic surface may be a surface provided with a hydrophilic coating, for example made in accordance with EP 0 093 093 and EP 0 217 771.

The catheter assembly may further comprise a wetting fluid, such as a wetting liquid, for wetting and activation of the hydrophilic surface. The wetting fluid may be arranged in a separate compartment within the assembly, to be released into the compartment housing the catheter shaft at a suitable time, such as immediately prior to use. However, the wetting fluid may also be provided within the same compartment as the catheter shaft, thereby maintaining the catheter in a wetted, activated state also during storage. This wetted state may be present immediately following closing and sealing of the package, or be obtained after some time of storage. The activated state may thus be provided by pre-wetting of the catheter, prior to arrangement of the catheter in the package, but may alternatively be provided after placement of the catheter in the package. Specifically, wetting of the hydrophilic surface may occur during a period of storage in a sealed container by provision of a humid atmosphere in the package, as is per se known in the art, e.g. by being arranged within a hydration element, such as a chamber or sachet which is liquid impermeable and vapor permeable.

Since the hydrophilic surface is then maintained in a wetted state during storage, the medical device is immediately ready-to-use upon removal from the package, and need not be wetted or treated in any way prior to use.

The package, formed by the casing and the cap sub-assembly, is preferably impermeable to the wetting fluid, and preferably made of a gas impermeable material. This ensures that moisture does not penetrate out from the package during storage, and enhances the shelf-life of the product.

The catheter is preferably a urinary catheter, and most preferably a urinary catheter for intermittent, short time use. The term "short term use" indicates a use that is limited in time, and in particular limited to a time period of less than 15 minutes, and preferably less than 10 minutes, and most preferably less than 5 minutes.

The wetting liquid is preferably an aqueous liquid, comprising at least 75% of weight of water, and preferably at least 80% of weight, and more preferably at least 85% of weight, and most preferably at least 90% of weight. In some embodiments, the wetting liquid may be plain water. However, the wetting liquid may also comprise one or more additives, such as an anti-bacterial agent, a pharmaceutical active substance, or the like.

According to another aspect of the invention there is provided a method for preparing a urinary catheter assembly as discussed above for use, the method comprising the steps:
remove the cap sub-assembly together with the catheter from the storage position in the casing;
rotate the catheter in relation to the cap sub-assembly by the joint; and
connect, releasably, the first engagement member of the casing with the second engagement member of the cap sub-assembly, whereby the catheter assumes an angularly displaced position, in which the longitudinal direction of the catheter is at an angle to the longitudinal direction of the casing and of the cap sub-assembly, the casing thereby serving as a laterally extending gripping member for the catheter.

These and other aspects of the invention will be apparent from and elucidated with reference to the embodiments described hereinafter.

### Brief description of the drawings

For exemplifying purposes, the invention will be described in closer detail in the following with reference to embodiments thereof illustrated in the attached drawings, wherein:
Figs. 1A - 1C are perspective views of a urinary catheter assembly in accordance with an embodiment of the present invention in a closed storage position (Fig. 1A), in an opened use position (Fig. 1C), and in an exploded view (Fig. 1B);
Figs. 2A - 2E are perspective views of a urinary catheter assembly in accordance with another embodiment of the present invention, the figures showing a closed storage position (Fig. 2A), an opened use position (Fig. 2E), various intermediate positions between these states (Figs. 2B-2D), and an illustration of the catheter assembly in use (Fig. 2F);
Figs. 3A - 3D show detailed views of a cap sub-assembly of a urinary catheter assembly in accordance with another embodiment of the present invention in various positions;
Figs. 4A - 4E are perspective views of a urinary catheter assembly in accordance with another embodiment of the present invention, the figures showing a closed storage position (Fig. 4A), an opened use position (Fig. 4D), and various intermediate positions between these states (Figs. 4B-4C); and
Figs. 5A - 5E are perspective views of a urinary catheter assembly in accordance with yet another embodiment of the present invention, the figures showing a closed storage position (Fig. 5A), an opened use position (Fig. 5E), and various intermediate positions between these states (Figs. 4B-4D).

### Detailed description of preferred embodiments

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length of the medical device, etc.

The urinary catheter assembly disclosed in relation to the illustrative example has an elongate, pen-like shape, and having a generally circular cross-section. However, as discussed in the foregoing the assembly may also have other shapes, such as being of a square, rectangular or oval shape.

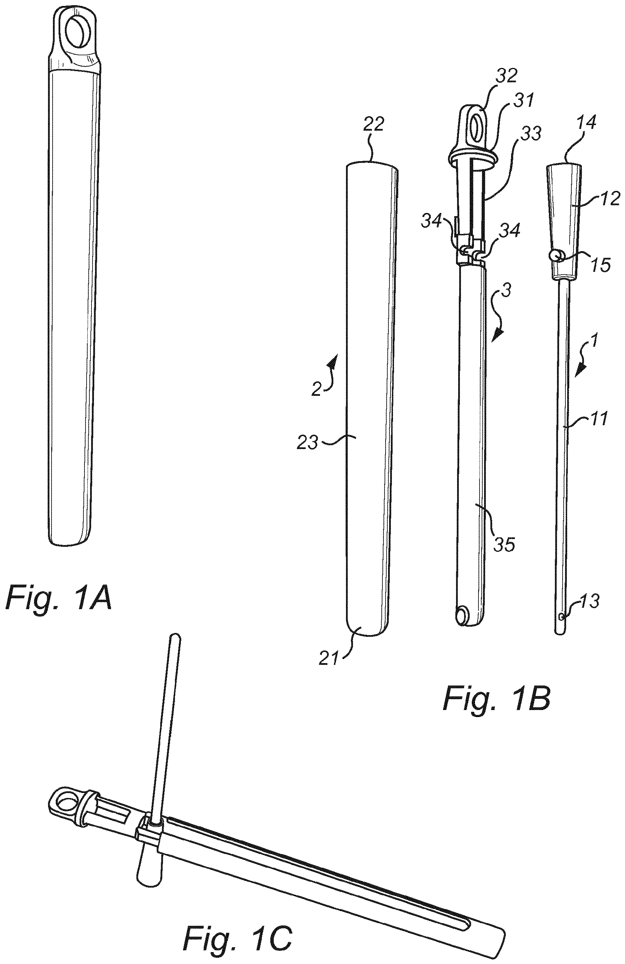

The catheter assembly as illustrated in Figs. 1A-1C comprises a catheter 1 having an insertable section 11, forming a catheter shaft, and a non-insertable section 12, forming a connector part. The non-insertable section 12 preferably has a larger diameter than the insertable section 11. The rear end of the non-insertable section may be flared or funnel-shaped, and may be arranged to be connected to a tapered connection part of a urine collection bag or the like. It may also be connected to an extension tube, or be used for direct discharge of the urine into a toilet bowl or the like.

At least a part of the insertable section 11 forms an insertable length to be inserted through a urethra of the user.

The insertable section comprises an insertion tip, which may be a closed, rounded end. Further the insertable section may comprise inlet openings 13, so called catheter eyes or eyelets, leading into a lumen extending through the catheter, and into a discharge outlet 14 arranged at the rearward end of the non-insertable section.

The insertable section 11 may comprise a hydrophilic surface, and form a hydrophilic catheter, as is per se well known in the art. The hydrophilic surface may be in the form of a hydrophilic surface coating, for example PVP, and which provides a low-friction surface when wetted with a wetting fluid. Typically, the insertable length is within 50-140 mm for a female patient and 200-350 mm for a male patient. Even though PVP is the preferred hydrophilic material, other hydrophilic materials may be used, such as hydrophilic polymers selected from polyvinyl compounds, polysaccharides, polyurethanes, polyacrylates or copolymers of vinyl compounds and acrylates or anhydrides, especially polyethyleneoxide, polyvinyl-pyrrolidone, heparin, dextran, xanthan gum, polyvinyl alcohol, hydroxy propyl cellulose, methyl cellulose, copolymer of vinylpyrrolidone and hydroxy ethylmethyl acrylate or copolymer of polymethylvinyl ether and maleinic acid anyhydride. However, instead of a hydrophilic surface coating, the entire insertable section of the catheter may be formed of a hydrophilic material.

However, alternatively the catheter may be non-hydrophilic, and may e.g. be lubricated with gel prior to insertion.

The assembly further comprises a casing 2 forming a tubular compartment which is closed at a proximal end 21 and open at the other, distal end 22. A tubular sidewall 23 extends between the ends. The casing is arranged to receive at least the insertable section 11 of the catheter, and preferably also a part of the non-insertable section 12, and to accommodate these parts of the catheter during storage. The tubular casing is arranged to be closed at the open distal end to form a sealed and sterile compartment for the accommodated catheter parts during storage. Closing and sealing of the open distal end 22 is obtained by a cap sub-assembly 3.

The cap sub-assembly comprises a cap 31, arranged to close and seal the casing. The cap consequently functions as a lid, sealing the assembly in the closed storage position. On the external side of the cap, a gripping member 32 may be provided. In the illustrative example, the gripping member is in the form of a ring, also allowing the catheter assembly to be hanged on a hanger or the like.

In addition, the cap sub-assembly functions as a holder and fixture for the catheter. To this end, the non-insertable, distal part of the catheter is pivotally connected to cap sub-assembly by a joint, such as a revolute joint. Hereby, the catheter and the cap sub-assembly can be aligned and in a straight disposition when in the closed storage position, and be angled in relation to each other, such as being perpendicular to each other, in the opened use position.

In one embodiment, the cap sub-assembly comprises an insert, connected to the cap 31, and extending inwards, to be inserted into the casing 2 in the closed storage position. The insert here comprises two parallel arms 33 extending downwardly from the cap 31. The arms comprise two oppositely arranged openings 34 arranged to receive two oppositely extending pins 15, extending outwardly from the distal end of the catheter. Hereby, a revolute joint is formed between the catheter and the cap sub-assembly. The openings may e.g. be in the form of semicircular openings, allowing the pins to be pressed into engagement.

The insert may further comprise an additional proximal part 35, extending further away from the cap, beneath the openings 34. This additional proximal part 35 may be shaped as a trough, accommodating the catheter shaft and the proximal part of the catheter therein. The additional proximal part provides additional stabilization and support to the catheter assembly, and also provides a connection between the cap sub-assembly and the casing, both, optionally, in the closed storage position, and in the opened use position, where the cap sub-assembly is semi-inserted into the casing.

Fig. 1A illustrates a closed storage position, in which the cap sub-assembly closes the casing, and in which the catheter is accommodated within the package formed by the cap sub-assembly and the casing. The catheter and the interior of the casing are preferably maintained sterile in this position.

For use of the catheter, the cap sub-assembly is loosened and removed from the casing, thereby extracting the catheter out from the casing. The catheter is then pivoted to an angular position, e.g. being 90 degrees in angle to the longitudinal direction of the cap sub-assembly. Thereafter, the proximal part of the cap sub-assembly is reinserted into the casing, into a semi-inserted state, as shown in Fig. 1C. In this position, the proximal part of the cap sub-assembly is connected to the casing, e.g. by a friction fit, and the casing functions as a handle for manipulation of the catheter during the catheterization, and in particular for a controlled and gentle insertion of the catheter into the meatus of the urethra.

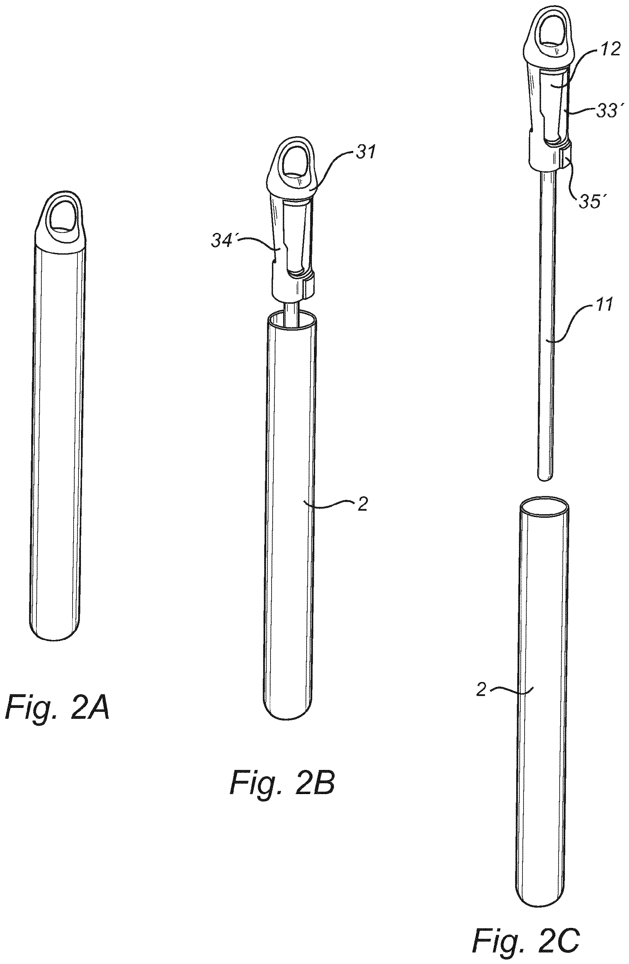

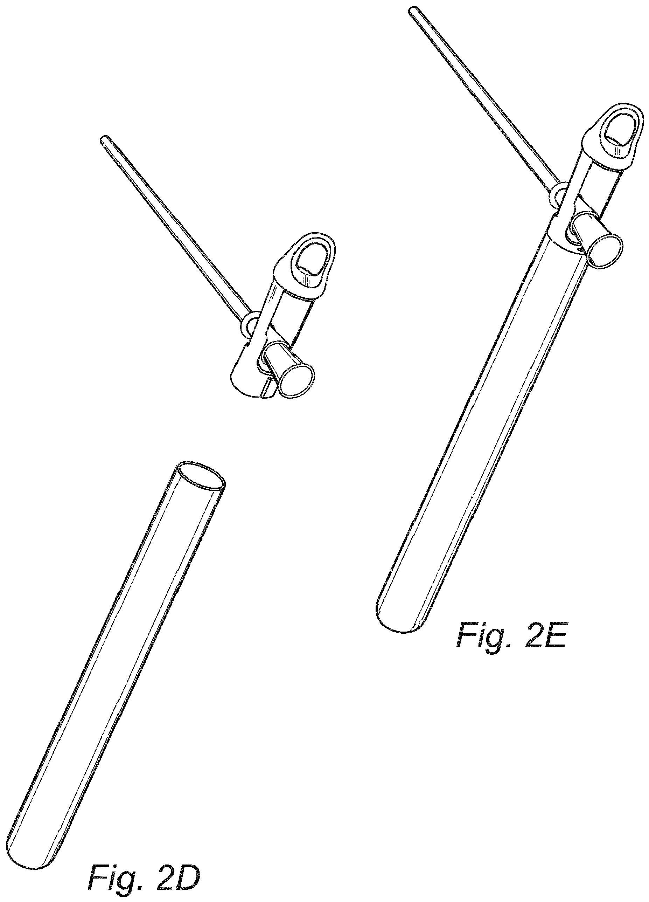

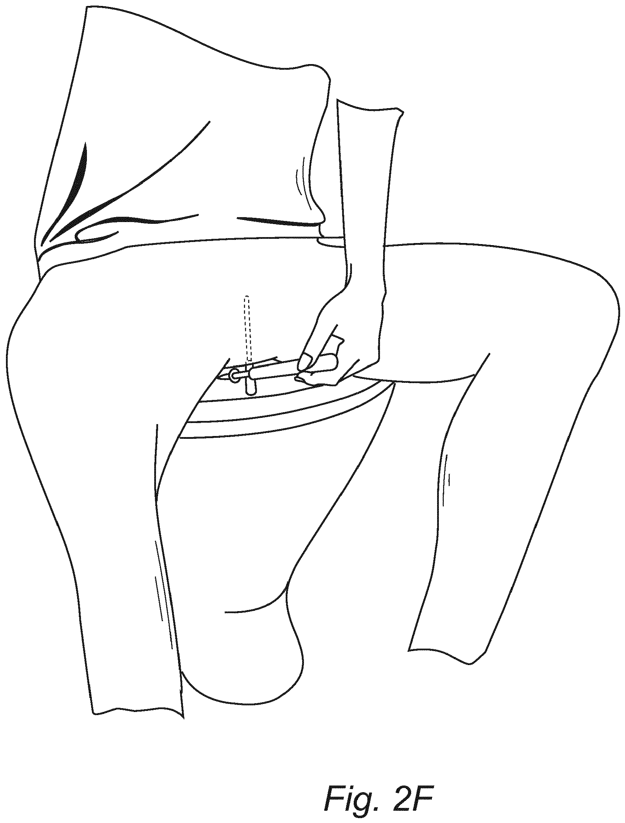

In another embodiment, illustrated in Fig. 2, the catheter assembly is similar to the embodiment discussed in relation to Fig. 1. However, here, the cap sub-assembly is realized in a somewhat different form, and also the revolute joint connecting the cap sub-assembly and the catheter is differently constituted.

In the embodiment of Fig. 2, the cap sub-assembly again comprises arms 33' extending downwardly from the cap 31. The arms are formed to be resilient, and are at a distal end connected by an encircling and overlapping proximal part 35'. The arms are further provided with a narrowing part 34', functioning as a revolute joint and a fixation for the catheter, arranged at a downward section, close to the overlapping proximal part 35'.

Fig. 2A illustrates the catheter assembly in a closed storage position.

For use, the cap sub-assembly is loosened from the casing, and removed, so that the catheter becomes exposed. This is, in sequence, illustrated in Figs. 2B and 2C.

The catheter is then pivoted into an angular state, e.g. being perpendicular to the cap sub-assembly, as illustrated in Fig. 2D. The distal part of the catheter is hereby inserted into the narrowing part 34' of the arms, thereby fixating the catheter in the angular position.

With the catheter in this angular state, the proximal part of the cap sub-assembly may be inserted into the casing again, whereby the overlapping resilient proximal part becomes slightly compressed, thereby locking the catheter in the angular position, and also firmly connecting the casing to the cap sub-assembly. This state is illustrated in Fig. 2E.

Fig. 2F illustrates the catheter assembly, when arranged in the use position, during actual use. As illustrated, the user may hold the handle in a pencil grip, and may easily maneuver the catheter in a very controlled way from a distance.

In the above-discussed embodiments, the cap is connected to the casing, in the closed storage position, by a friction fit. However, alternative connections are also feasible, such as by threaded connections, bayonet connections, welded or adhesive breakable connections, and the like. In the following, an embodiment with a threaded connection will be discussed in some more detail.

Further, the connection between the cap sub-assembly and the casing in the open use position may be realized in various ways, such as by a friction fit, a threaded connection, etc. In the embodiment to be discussed in the following, a snap-lock arrangement is used.

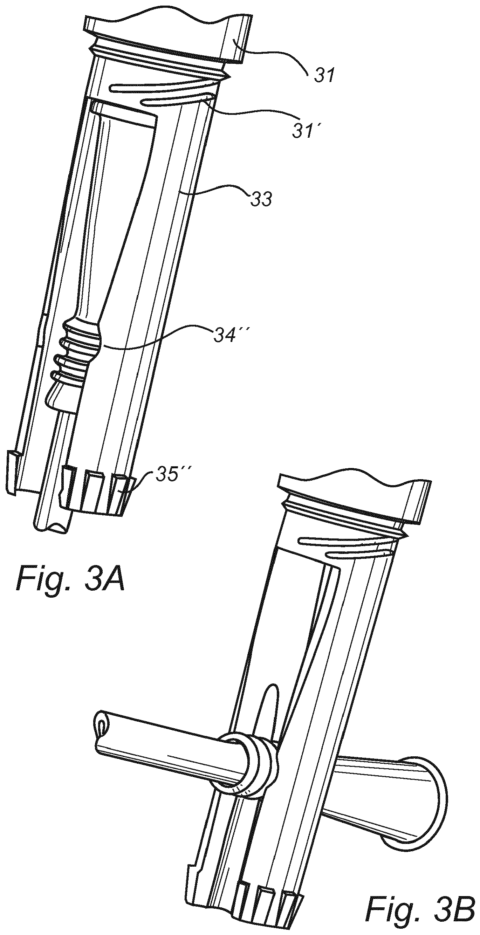

In the embodiment of Fig. 3A-3D, the cap sub-assembly is provided with external threads 31', arranged in the vicinity of the cap 31. The threads 31' are arranged to mate with corresponding interior threads provided at the vicinity of the distal end of the casing 25. Hereby, a strong connection is formed, which is still easy to release by twisting the cap in relation to the casing.

Further, the proximal part of the cap sub-assembly is here provided with notches 35" arranged to form a snap-lock connection with corresponding protrusions 24 in the interior wall of the casing. Hereby, a strong connection is formed between the cap sub-assembly and the casing in the use position, which is yet easily releasable when catheterization is finished.

In this embodiment, the revolute joint is further formed by relatively narrow arms 31, provided with notches 34" arranged to firmly receive the distal part of the catheter when in the angular use position.

Fig. 3A illustrates the catheter and the cap sub-assembly in a closed storage position, wherein the catheter is aligned with the cap sub-assembly. Fig. 3B illustrates the catheter and the cap sub-assembly in the angular use position.

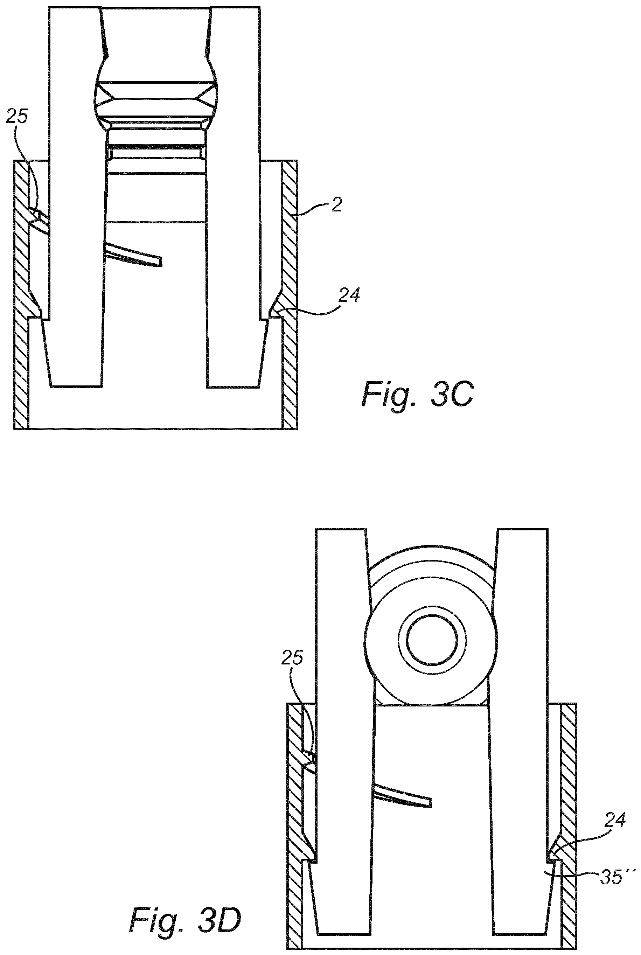

Fig. 3C illustrates the catheter and the catheter sub-assembly when inserted into the casing 2, in a partly removed state.

Fig. 3D illustrates the catheter and the catheter sub-assembly when reinserted into the casing in the use position, wherein the notches 35" form a snap-lock connection with the protrusions 24.

In the previously discussed embodiment, the casing forms a first engagement member at the internal surface of the casing, in the vicinity of the open end, and the proximal, forward part of the cap sub-assembly forms the second engagement member. Thus, in these embodiments, the cap sub-assembly is reinserted into the casing to adopt the use position. The engagement may be maintained by a friction fit. However, it is also feasible to provide the first engagement member on another position on the casing, such as at the closed end. Alternatively, or additionally, it is feasible to provide the second engagement member on another position on the cap sub-assembly, such as at the external, distal end. Some embodiments of such realizations will be discussed in the following.

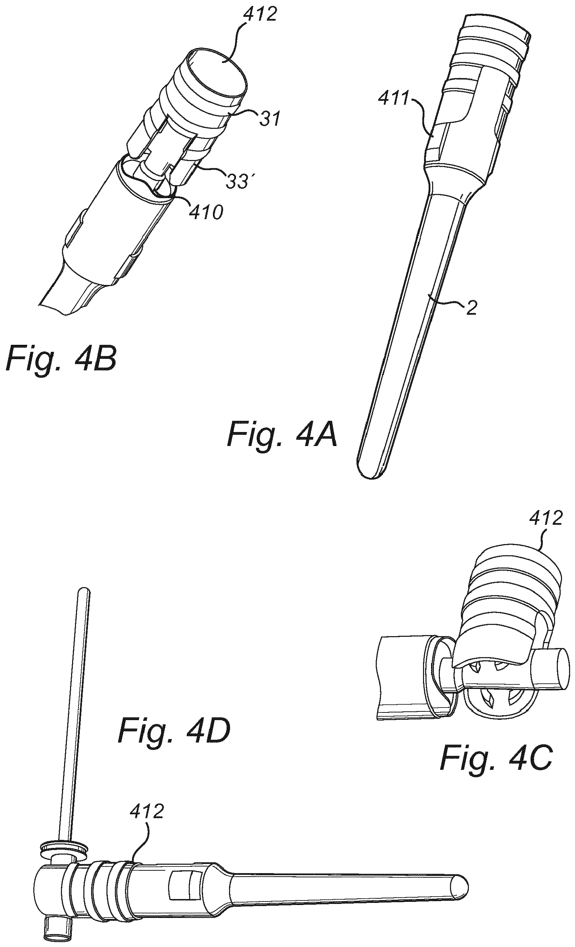

In the embodiment of Figs. 4A-4D, the cap sub-assembly is arranged exteriorly of the casing, with the arms 33' extending on the outside of the casing 2 instead of on the inside. The cap sub-assembly is connected to the catheter by a revolute joint, here comprising protruding pins arranged on the distal part of the catheter, and being rotatably engaged by openings in the cap sub-assembly, similar to what has been discussed in relation to other embodiments.

The rim 410 of the open end of the casing 2 may have a non-planar, slanted or curved shape, and the cap sub-assembly may have a corresponding shape in the interface towards the rim, when arranged in the closed storage position. This facilitates removal of the cap sub-assembly, since twisting of the cap sub-assembly in relation to the casing will provide a force in the longitudinal direction separating the cap sub-assembly from the casing. For further facilitation of the opening, the cap sub-assembly and/or the casing may be provided with protruding grips 411, making the twisting simpler.

In this embodiment, the first engagement member is formed on the exterior side of the casing, at or in the vicinity of the open end. Further, in this embodiment, the second engagement member is formed at the distal part of the cap sub-assembly, i.e. in the part being opposed to the catheter. The second engagement member may e.g. be provided in the form of an opening 412, arranged at the distal end of the cap sub-assembly, allowing the open end of the casing to be inserted into the opening 412 of the cap sub-assembly. The engagement may here be obtained only by friction, but additionally, threads or the like may be provided to enhance the connection.

In Fig. 4A, the catheter assembly of this embodiment is illustrated in its closed storage position. In preparation for use, the cap sub-assembly is twisted, thereby separating the cap sub-assembly from the casing, as illustrated in Fig. 4B. The cap sub-assembly may then be rotated in relation to the catheter even prior to full extraction of the catheter from the casing, as illustrated in Fig. 4C. After extraction of the catheter, the rear opening of the cap sub-assembly is mounted on top of the open end of the casing, as illustrated in Fig. 4D.

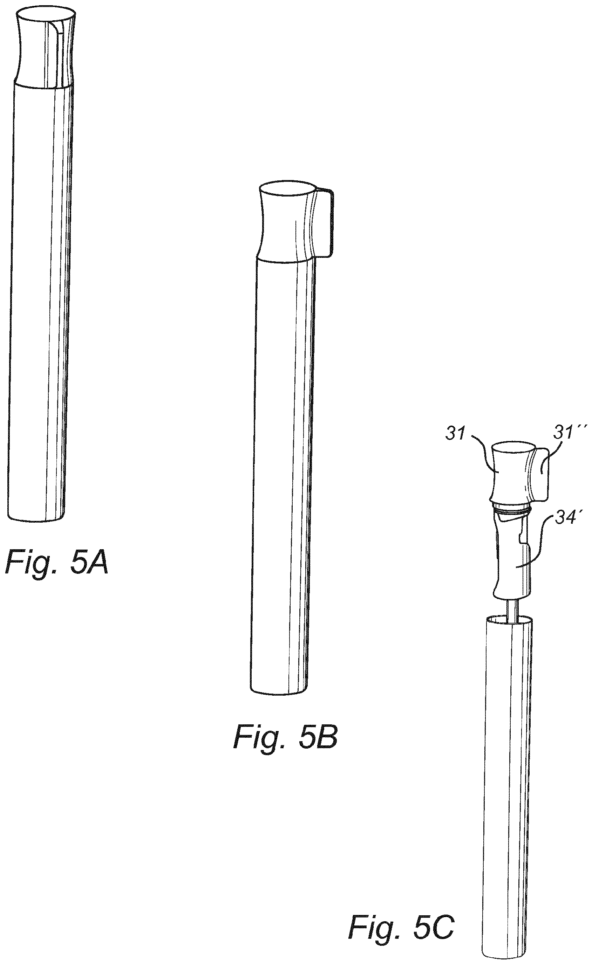

With reference to Figs. 5A-5E, yet another embodiment of the catheter assembly will be discussed. This embodiment is in most parts and aspects similar to previously discussed embodiments, apart from the differences discussed in the following.

In this embodiment, the cap sub-assembly comprises a cap 31, here provided with an optional extending grip 31", to facilitate twisting. The cap is maintained in a closed position by a friction fit, and a gasket, O-ring or the like may be provided in the interface, to ensure tight sealing and sterility.

The cap sub-assembly further comprises a downwardly extending proximal part, arranged to be accommodated in the casing in the closed storage position. The catheter and the cap sub-assembly are, similar to previously discussed embodiments, provided with a revolute joint, allowing the catheter to be pivoted in relation to the cap sub-assembly.

In this embodiment, the second engagement member is provided in the proximal part of the cap sub-assembly, similar to the embodiment discussed in relation to Figs. 1 and 2. However, here the first engagement member is provided at the proximal side of the casing, instead of at the distal side. To this end, an opening 510 may be formed at the proximal end of the casing, and optionally provided with a protrusion 511 arranged within the opening 510. The protrusion may e.g. be slightly conical. Hereby, an opening formed as an annular recess is provided. Hereby, the inserted part of the cap sub-assembly will be held more firmly in place, by the friction working on both the internal and external side thereof.

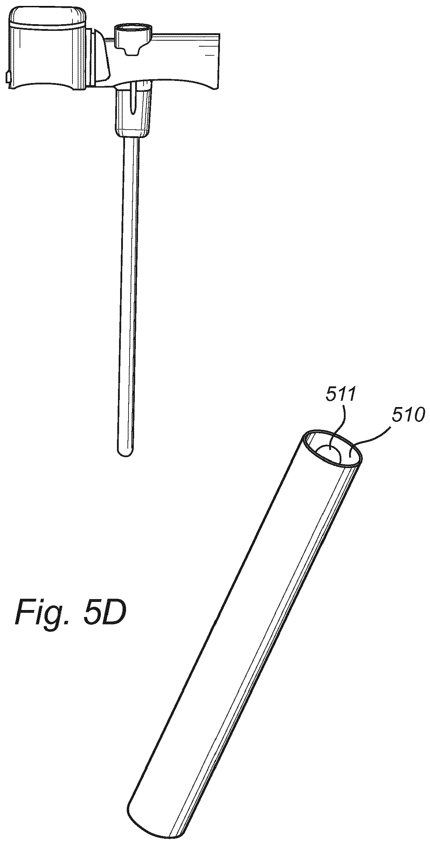

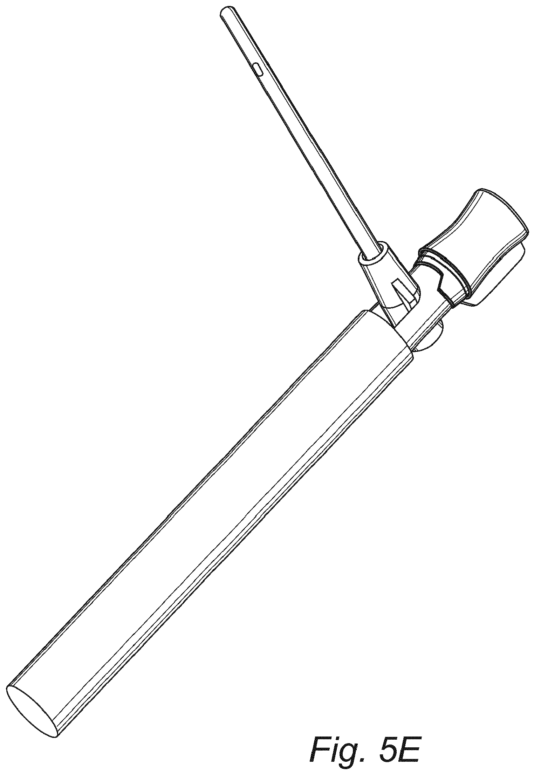

Figs. 5A and 5B illustrate the catheter assembly in a closed storage position from two different views. Fig. 5C illustrates the assembly when the cap sub-assembly has been twisted open, and partly extended out from the casing. Fig. 5D illustrate the assembly in a state where the cap sub-assembly has been removed from the casing, and the catheter has been pivoted in relation to the cap sub-assembly. Fig. 5E illustrate the catheter assembly where the cap sub-assembly has been mounted in a use position.

In all the embodiments discussed above, the cap sub-assembly is arranged to form a releasable connection with the casing, and a pivotable connection with the catheter. To maintain the cap in a closed storage position, a mechanical connection may be formed by threads or the like, engaging with corresponding threads on the casing, to form a twist-off cap. However, the cap may also be connected by a friction connection, a friction fit, as shown in some of the illustrative examples.

Both the casing and the cap sub-assembly may be formed by a relatively rigid or hard plastic material, but the cap sub-assembly may alternatively be formed by a less rigid and more flexible material.

Since the casing and the cap sub-assembly forms a sealed and sterile compartment for the catheter, thereby maintaining the catheter clean and sterile during storage, no additional packages or the like are needed to maintain the assembly in a clean and sterile condition during storage and prior to use.

The tubular case is here illustrated as being essentially circular in cross-section, but other shapes, such as a rectangular or oval shape, may also be contemplated.

The second engagement member may be arranged at the closed end of the tubular case or at the open end of the tubular case. Similarly, the first engagement member may be formed at the proximal end of the cap sub-assembly, or at the distal end. The second engagement member preferably has a form and shape complementary to the form and shape of the first engagement member, thereby allowing the first and second engagement member to mate and connect to each other. The first and second engagement members may be engaged only by friction, or by threads, bayonet couplings. Preferably, the first and second engagement members may be connectable through one of threads, a friction fit, a snap-lock and a snap-fit arrangement, and the first and second engagement members are preferably complementary to each other in shape and/or size, thereby enabling engagement between the members. Many other alternatives for engaging the first and second engagement member are naturally feasible, as would be appreciated by the skilled reader.

It is also feasible that the first and second engagement members are such that engagement in more than one way is possible, e.g. allowing the tubular case to be engaged in two or more different angular positions in relation to the cap-sub-assembly and the catheter. It is also possible to provide two or more first engagement members on the casing, and/or two or more second engagement members on the cap sub-assembly.

During catheterization, the urine may be drained directly from the outlet opening of the catheter and into a toilet bowl. However, it is also possible to connect an extension tube or urine collection bag to the funnel shaped outlet opening.

After catheterization, the catheter can be reassembled into the closed storage position, essentially by performing the preparation steps in the reverse order. Once the catheter is reinserted into the tubular case and the cap is reclosed, the package will retain all or at least some of its original sealing qualities, and will consequently not leak and protect the user from spillage and contamination.

A label that breaks upon opening, or other types of tamper proof, may be provided to make it easy to see if a urinary catheter assembly has been opened or used.

For producing and assembling the urinary catheter assembly, the cap sub-assembly, the casing, and the catheter may each be produced and provided separately.

The catheter can e.g. be produced in a conventional way, using conventional manufacturing equipment. The catheter can be produced by any biocompatible polymeric material having sufficient stiffness and flexibility, as is per se well known in the art.

The casing is preferably formed by relatively hard plastic material, such as HDPE, nylon or polypropylene, and may e.g. be produced by injection molding. The casing may further be produced by a non-transparent and opaque material, such that the contents cannot be easily identified.

The cap sub-assembly may be produced by the same or similar material as the casing, or may alternatively be formed of a relatively less hard and more flexible material, and may also be produced e.g. by injection molding.

When assembled, the assembly is sterilized and ready for use.

In case a hydrophilic catheter is used, the urinary catheter assembly may also comprise a wetting fluid. However, the wetting fluid for activation of the catheter need not be provided within the package. Instead, a wetting fluid may be poured into the package after opening of the package, for wetting of the catheter while it still remains in the package. In some occasions, the catheter may even be removed from the package and wetted e.g. in a different container, even though this is normally not preferred.

However, preferably the wetting fluid is arranged within the assembly, and preferably within the casing, so that the hydrophilic surface of the catheter can be activated even before opening of the package. In one embodiment, the wetting fluid is arranged separated from the catheter, in a wetting fluid container (not shown), such as a pouch or a sachet. The wetting fluid container is openable by means of e.g. exerting a pressure to the container, whereby the wetting fluid is released into the package, thereby wetting the hydrophilic surface of the catheter. The wetting fluid container may also be arranged to open automatically when the catheter assembly is opened, e.g. by the opening of the cap. In another embodiment, the wetting fluid is arranged directly in the compartment of the casing accommodating the catheter, so that the hydrophilic surface of the catheter is in direct contact with the wetting fluid during storage, and thereby is maintained in an activated, ready-to-use state.

The wetting fluid is preferably a liquid, and most preferably an aqueous liquid, such as water or saline. However, the wetting fluid may also be a gas, providing a moist atmosphere in the package sufficient for activation of the hydrophilic surface. Thus, the wetting fluid may be any fluid, gas or liquid, that wets/activates a hydrophilic surface of the catheter.

In case a non-hydrophilic catheter is used, the urinary catheter assembly may comprise a supply of lubricant, such as a compartment arranged overlying the catheter shaft, so that the shaft is lubricated while being pulled out from the tubular case.

Specific embodiments of the invention have now been described. However, several alternatives are possible, as would be apparent for someone skilled in the art. For example, although the wetting fluid in the described embodiments is arranged in direct contact with the catheter, but may alternatively be arranged separated from the catheter, in a wetting fluid container. Further, it is possible to use many different types of engagement members for attaching the casing to the cap sub-assembly for use as a handle. The cap sub-assembly may similarly be designed and shaped in various ways. Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. A catheter assembly comprising:
a casing having a tubular compartment which is closed at one end and open at the other end;
a cap sub-assembly;
a catheter having a catheter shaft, a proximal insertion end and a distal part having a distal end, wherein a flow path is defined between the proximal end and the distal end, and wherein the distal part is pivotally connected to the cap sub-assembly by a joint, and preferably a revolute joint;
wherein, in a closed storage position, the catheter shaft is accommodated in the tubular compartment and the cap sub-assembly seals the open end of the tubular compartment, wherein the catheter shaft extends in a longitudinal direction aligned with a longitudinal direction of the casing and of the cap sub-assembly; and
wherein the casing is provided with a first engagement member, and the cap sub-assembly is provided with a second engagement member, whereby the cap sub-assembly, in an open use position, is attachable to the casing with the catheter in an angularly displaced position, in which the longitudinal direction of the catheter is at an angle to the longitudinal direction of the casing and of the cap sub-assembly, the casing thereby serving as a laterally extending gripping member for the catheter.

2. The catheter assembly of claim 1, wherein the second engagement member is provided on an end of the cap sub-assembly being inserted into the casing in the closed position.

3. The catheter assembly of claim 1, wherein the second engagement member is provided on an end of the cap sub-assembly being opposite to the casing in the closed position.

4. The catheter assembly of any one of the preceding claims, wherein the first engagement member is provided in the vicinity of the open end of the casing.

5. The catheter assembly of claim 4, wherein the first engagement member is provided on an interior surface in the vicinity of the open end of the casing.

6. The catheter assembly of claim 4, wherein the first engagement member is provided on an external surface in the vicinity of the open end of the casing.

7. The catheter assembly of any one of the preceding claims, wherein the first engagement member is provided in the vicinity of the closed end of the casing.

8. The catheter assembly of any one of the preceding claims, wherein the joint is a revolute joint formed by laterally protruding pins arranged on the distal part of the catheter and corresponding openings for accommodation of the protruding pins on the cap sub-assembly.

9. The catheter assembly of any one of the preceding claims, wherein the cap sub-assembly comprises a head portion providing a sealed closure of the case, and an insertable part being insertable into the casing, the insertable part being pivotally connected to the distal part of the catheter.

10. The catheter assembly of any one of the preceding claims, wherein the first and second engagement members are connectable through one of threads, a friction fit, a bayonet mount, a Luer connection, a snap-lock and a snap-fit arrangement.

11. The catheter assembly of any one of the preceding claims, wherein the catheter is a urinary catheter, and preferably an intermittent urinary catheter.

12. The catheter assembly of any one of the preceding claims, wherein the catheter is a hydrophilic catheter, and wherein the assembly further comprises a wetting fluid for activation of the hydrophilic catheter.

13. The catheter assembly of any one of the preceding claims, wherein the distal part of the catheter comprises a flared connector.

14. The catheter assembly of any one of the preceding claims, wherein the catheter in the open use position is at an angle to the longitudinal direction of the casing and cap sub-assembly in the range of 60-120 degrees, and most preferably 75-105 degrees.

15. The catheter assembly of any one of the preceding claims, wherein the casing together with the cab sub-assembly provides a sterile and moisture proof compartment for the insertable section of the catheter in the closed storage position.

16. Method for preparing a urinary catheter assembly in accordance with any one of the claims 1-15 for use, the method comprising the steps:
remove the cap sub-assembly together with the catheter from the storage position in the casing;
rotate the catheter in relation to the cap sub-assembly by the joint; and
connect, releasably, the first engagement member of the casing with the second engagement member of the cap sub-assembly, whereby the catheter assumes an angularly displaced position, in which the longitudinal direction of the catheter is at an angle to the longitudinal direction of the casing and of the cap sub-assembly, the casing thereby serving as a laterally extending gripping member for the catheter.
